# EUROPEAN PATENT APPLICATION

(11) **EP 1 123 702 A1**
(43) Date of publication of application: **16.08.2001**
(21) Application number: 99947968.6
(22) Date of filing: 19.10.1999
(51) Int. Cl.: A61K 31/445, A61K 31/47

(54) **ANALGESICS**

(30) Priority: 19.10.1998 JP 29668198
(71) Applicant: Eisai Co., Ltd., Tokyo 112-8088 (JP)
(72) Inventor: UENO, Kohshi, Tsukuba-shi, Ibaraki 305-0042 (JP); SASAKI, Atsushi, Tsukuba-shi, Ibaraki 305-0035 (JP); KITAZAWA, Noritaka, Toride-shi, Ibaraki 302-0004 (JP); KAWANO, Koki, Tsukuba-shi, Ibaraki 305-0045 (JP); OKABE, Tadashi, Famiru Mitsuho A201, Tsuchiura-shi, Ibaraki 300-0034 (JP); TAKAHASHI, Keiko, Ushiku-shi, Ibaraki 300-1222 (JP); MATSUNAGA, Manabu, Tsukuba-shi, Ibaraki 305-0003 (JP); YASHIKAWA, Yukie, Ushiku-shi, Ibaraki 300-1234 (JP)
(74) Representative: HOFFMANN - EITLE
(86) International application number: JP9905761
(87) International publication number: WO0023075

(57) **Abstract**

The present invention provides a novel analgesic useful for various diseases such as headache and migraine, and to pain and ache accompanied by trauma, physical pressure, etc. Further, the present invention is an analgesic comprising the benzene compound (I) represented by the following formula: 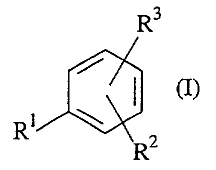 wherein, R¹ is a group represented by the following formula: 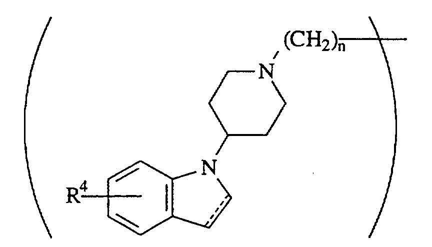 or a group represented by the following formula: 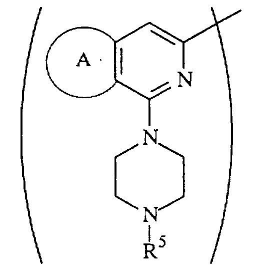 R², R³, R⁴ and R⁵ are substituents; and the ring A is benzene ring or thiophene ring, or a pharmacologically acceptable salt thereof as an active ingredient.

## Description

### Field of the Invention

The present invention relates to a novel analgesic.

### Prior Art

It is common that various diseases such as headache and migraine and pain and ache accompanied by trauma and physical pressure continue for a certain period and, although being dependent upon the degree, they are intolerable symptoms. Accordingly, aside from the therapy for causative diseases, relief of pain and ache greatly contributes to an improvement in quality of life of the patient.

At present, antipyretic/analgesic/anti-inflammatory agents or narcotics are utilized as analgesics to those pain and ache.

To be more specific, aspirin, ibuprofen, indomethacin, piroxicam, mefenamic acid etc. are widely used as antipyretic/analgesic/anti-inflammatory agents, while morphine hydrochloride and morphine sulfate are mostly utilized as narcotics.

However, all of the antipyretic/analgesic/antiinflammatory agents as mentioned above are the drugs based upon a suppressive action to biosynthesis of prostaglandin and, therefore, there is a problem that the frequency of damaging the digestive organs as an adverse action is extremely high. In addition, aspirin etc. have an adverse action of worsening the asthma.

On the other hand, since narcotics have a problem of drug dependency, a careful control for the administration is necessary and, moreover, adverse actions such as constipation are frequent.

Therefore, actually, the present situation is that no analgesics which show a useful effect for the therapy or improvement in various diseases such as headache and migraine as well as pain and ache accompanied by trauma and physical pressure, and which have an excellent safety have been available, whereby there has been a strong demand for the development of novel medicaments.

### Disclosure of the Invention

In order to improve such a present situation, the present inventors have carried out investigations and studies for many years.

As a result, it has been unexpectedly found that the benzene compounds (I) in accordance with the present invention have the same as or even more analgesic effect than narcotics and have a high safety as well and accordingly they are able to achieve the desired object whereupon the present invention has been accomplished.

Accordingly, an object of the present invention is to provide a novel agent for treating or improving various diseases such as headache and migraine and to pain and ache accompanied by trauma, physical pressure etc. for which no highly clinically useful pharmaceutical agents have been available, which has a high clinical usefulness and an excellent safety.

Further objects are to provide a method for preventing, treating or improving headache and migraine, by administering a pharmacologically effective dose of the benzene compound (I) of the present invention or a pharmacologically acceptable salt thereof to a person, and also to provide a use of the benzene compound (I) or a pharmacologically acceptable salt thereof for producing analgesics.

Herein, the benzene compound (I) which is an active ingredient in the present invention is a compound represented by the following formula: 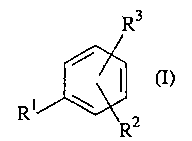 wherein, R¹ is a group represented by the following formula: 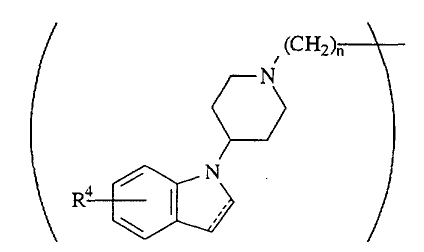 (wherein, R⁴ is a group selected from a lower acylaminoalkyl group, an amido lower alkyl group, an N-lower alkylamidoalkyl group, an N,N-di-lower alkylamidoalkyl group and an N-hydroxy lower alkylamidoalkyl group; n is an integer of 0 or 1 to 3; and the bond ----- means a single or double bond) or a group represented by the following formula: 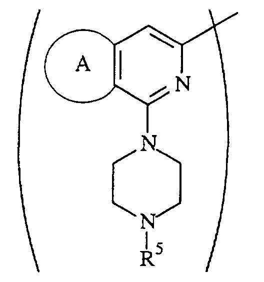 (wherein, the ring A is benzene ring or thiophene ring; and R⁵ is a lower alkyl group or a hydroxy lower alkyl group); and R² and R³ are the same as or different from each other and each is a group selected from hydrogen atom, halogen atom, a lower alkyl group, a lower alkoxy group, cyano group, a hydroxy lower alkyl group, a hydroxy lower alkoxy group, an N-lower alkylamide group and a lower alkylsulfonylaminoalkyl group, or a pharmacologically acceptable salt thereof.

Specific examples of the above lower alkyl group are linear or branched alkyl groups having 1-6 carbons such as methyl group, ethyl group, n-propyl group, isopropyl group, n-butyl group, isobutyl group, tert-butyl group, pentyl group and hexyl group.

Specific examples of the halogen atom are fluorine atom, chlorine atom and bromine atom and, more preferably, fluorine atom or chlorine atom.

Specific examples of the lower alkoxy group are the groups such as methoxy group, ethoxy group, n-propoxy group, isopropoxy group, n-butoxy group, isobutoxy group, pentyloxy group and hexyloxy group, where oxygen atom is bonded to the above-mentioned lower alkyl groups.

In the benzene compound (I) according to the present invention, optical isomers, geometrical isomers, hydrates or polycrystalline may be present and it goes without saying that all of them are included in the present invention.

Incidentally, with regard to the pharmacologically acceptable salt in present invention, there is no particular limitation so far as an addition salt is formed with the benzene compound (I) and, usually, mineral acid addition salts such as hydrochloride, sulfate, nitrate and phosphate; organic acid addition salts such as oxalate, maleate and fumarate; and sulfonic acid addition salts such as methanesulfonate, benzenesulfonate and toluenesulfonate are exemplified.

With regard to the benzene compound (I), the compound represented by the following formula (II) or (III): 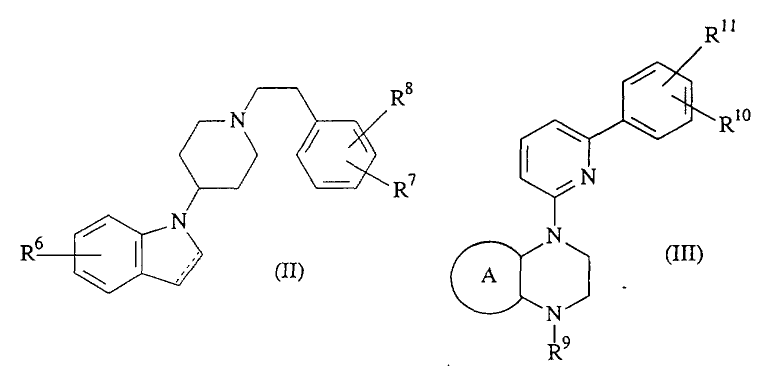 (wherein, R⁶ has the same meaning as the above-mentioned R⁴; and R⁷, R⁸, R¹⁰ and R¹¹ have the same meanings as the above-mentioned R² and R³; the ring A has the same meaning as mentioned above; and the bond ----- means a single or double bond), or a pharmacologically acceptable salt thereof is more preferably exemplified.

More specifically, the following compounds are proposed as the benzene compound (I) according to the present invention, although the present invention is not limited to them.
(1) 1-[1-(4-Fluorophenethyl)piperidin-4-yl]-6-(N-acetylaminomethyl)indole;
(2) 1-[1-(4-fluorophenethyl)piperidin-4-yl]-6-(N-propionylaminomethyl)indole;
(3) 1-[1-(4-methoxyphenethyl)piperidin-4-yl]-6-(N-acetylaminomethyl)indole;
(4) 1-[1-(2-fluorophenethyl)piperidin-4-yl]-6-(N-acetylaminomethyl)indole;
(5) 1-[1-(4-fluorophenethyl)piperidin-4-yl]-6-(N-acetylamidemethyl)indole;
(6) 1-[1-(4-fluorophenethyl)piperidin-4-yl]-6-(N-hydroxyethylamidemethyl)indole;
(7) 1-[1-(4-fluorophenethyl)piperidin-4-yl]-6-(N,N-dimethylamidemethyl)indole;
(8) 1-[1-(4-fluorophenethyl)piperidin-4-yl]-6-amidemethylindole;
(9) 1-[1-(4-fluorophenethyl)piperidin-4-yl]-6-(N-methylamidemethyl)indole;
(10) 1-[1-(2-fluorophenethyl)piperidin-4-yl]-6-(N-methylamidemethyl)indole;
(11) 1-[1-(2-fluorophenethyl)piperidin-4-yl]-6-(N-ethylamidemethyl)indole;
(12) 1-[1-(2-fluorophenethyl)piperidin-4-yl]-6-(N-hydroxyethylamidemethyl)indole;
(13) 1-[1-(2-fluorophenethyl)piperidin-4-yl]-6-amidemethylindole;
(14) 1-[1-(4-fluorophenethyl)piperidin-4-yl]-6-(N-acetylamidemethyl)indoline;
(15) 1-(4-ethylpiperazin-1-yl)-3-(4-methoxyphenyl)isoquinoline;
(16) 1-(4-ethylpiperazin-1-yl)-3-(4-hydroxyethoxyphenyl)isoquinoline;
(17) 1-(4-ethylpiperazin-1-yl)-3-[4-(1-hydroxypropyl)phenyl]isoquinoline;
(18) 1- (4-ethylpiperazin-1-yl)-3-[4-(N-propylamide)phenyl]isoquinoline;
(19) 1-(4-ethylpiperazin-1-yl)-3-[3-fluoro-4-(3-hydroxy-3-methylbutyl)phenyl]isoquinoline;
(20) 1-(4-ethylpiperazin-1-yl)-3-(3-methoxy-4-hydroxypropylphenyl)isoquinoline;
(21) 1-(4-ethylpiperazin-1-yl)-3-(3-cyano-4-hydroxyethoxyphenyl)isoquinoline;
(22) 1-(4-ethylpiperazin-1-yl)-3-(3-hydroxypropylphenyl)isoquinoline;
(23) 1-(4-ethylpiperazin-1-yl)-3-(3-chloro-4-propylsulfonylaminomethylphenyl)isoquinoline;
(24) 4-(4-ethylpiperazin-1-yl)-6-[4-(2-hydroxypropoxy)phenyl]thieno[3,2-c]pyridine;
(25) 4-(4-ethylpiperazin-1-yl)-6-[4-(2-hydroxy-2-methylpropoxy)phenyl]thieno[3,2-c]pyridine;
(26) 4-(4-ethylpiperazin-1-yl)-6-[4-(1-hydroxyethyl)phenyl]thieno[3,2-c]pyridine; and
(27) 4- (4-hydroxyethylpiperazin-1-yl)-6-[4-(2-hydroxy-2-methylpropoxy)phenyl]thieno[3,2-c]pyridine.

In order to more specifically show those compound examples, their structural formulae will be given as follows.

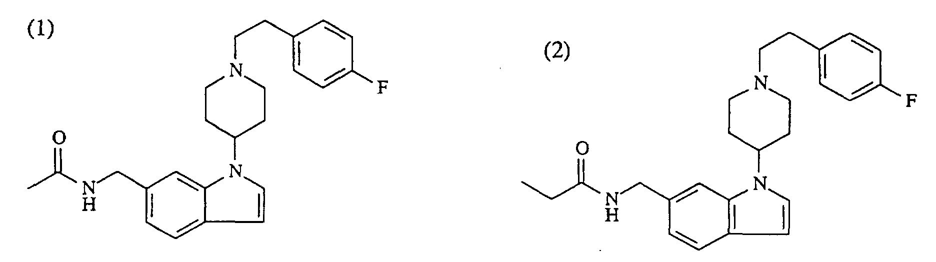

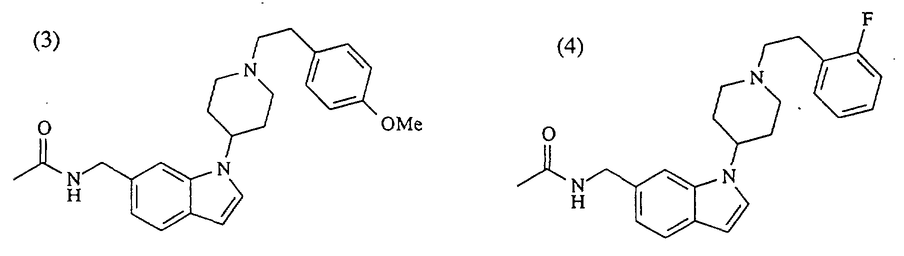

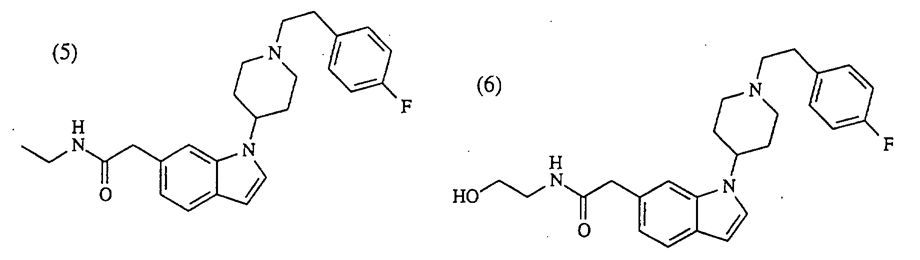

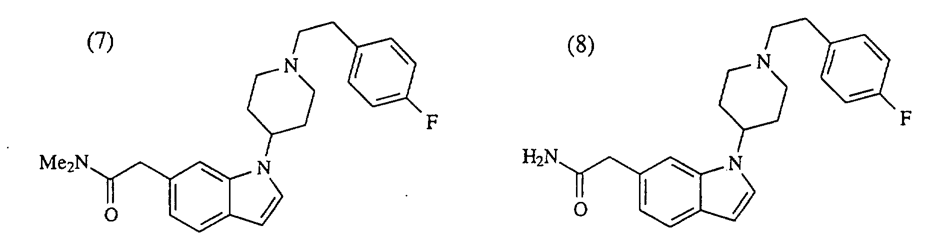

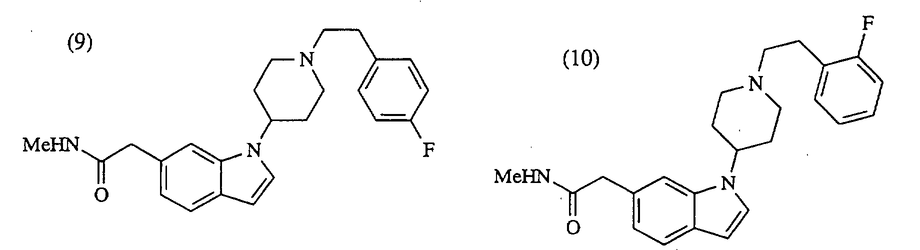

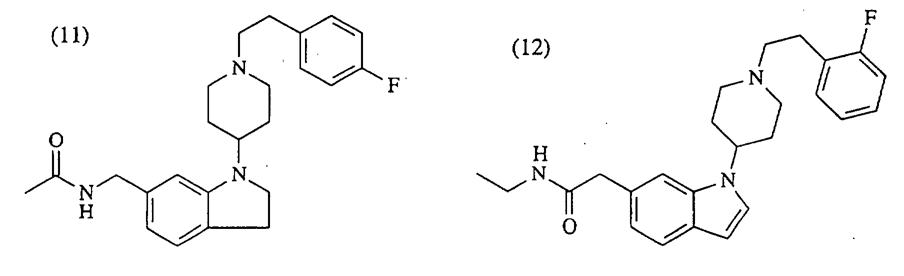

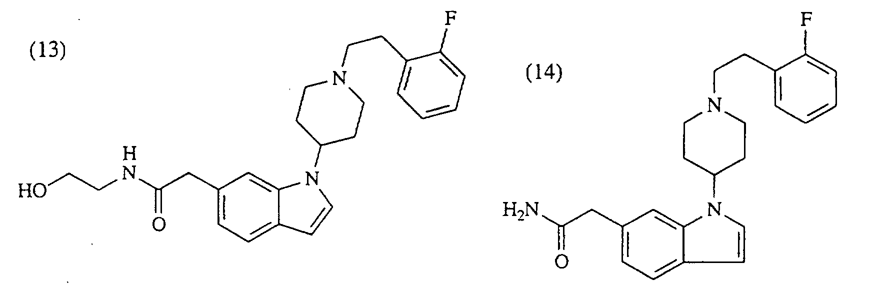

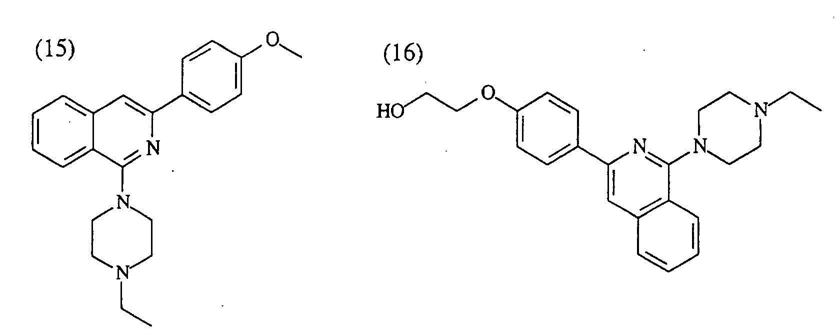

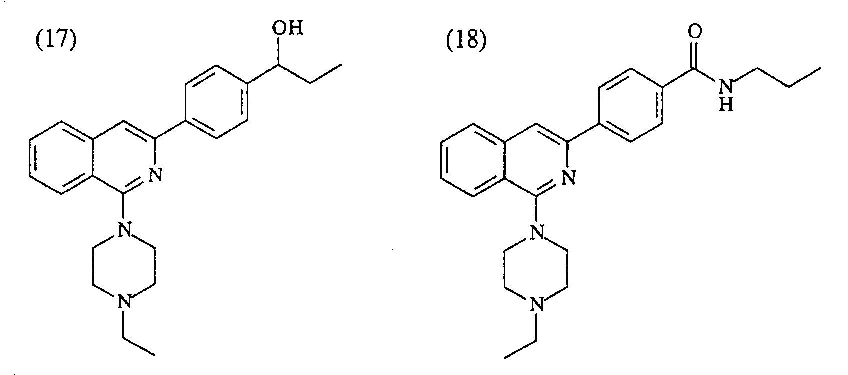

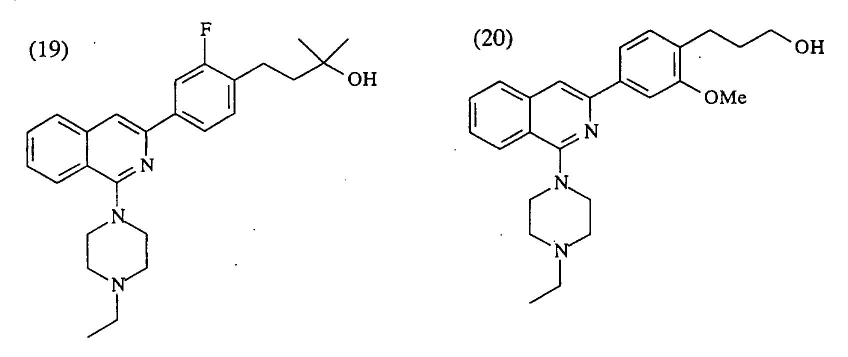

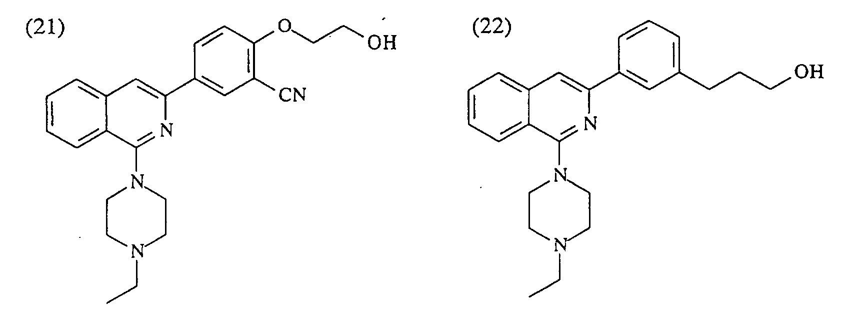

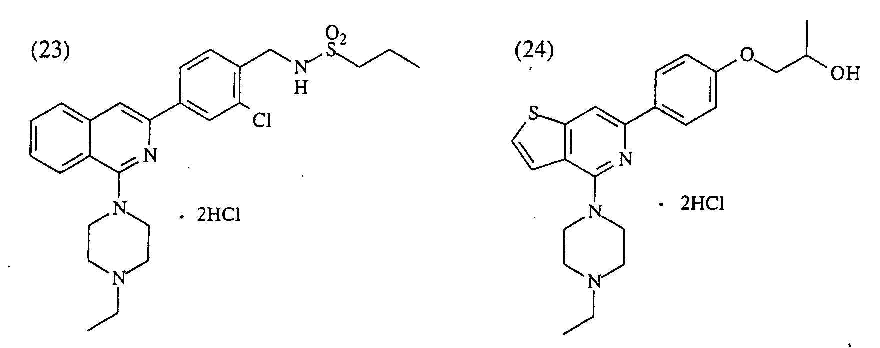

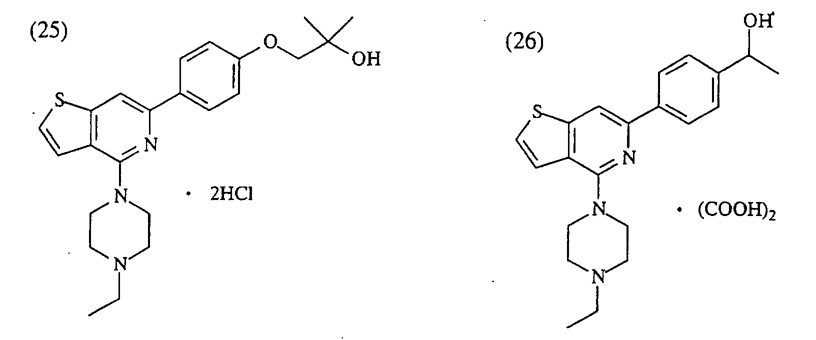

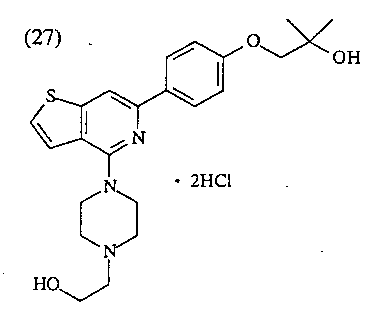

Incidentally, the benzene compounds (I) according to the present invention are known compounds and can be synthesized by the method mentioned in WO98/43956 or WO99/18077.

Now, the analgesic effect of the benzene compound (I) according to the present invention will be specifically shown by means of pharmacological experimental examples by a writhing method using acetic acid.

### Pharmacological Experimental Examples

### (1) Animals used

Male mice of a ddY strain of 4-7 weeks age were used. The mice were subjected to the experiment after a preliminary breeding for more than one week. During the breeding period, the mice were made free to take a solid feed and sterilized tap water and, from the evening of the day before the experiment, they were fasted but were made free to take water.

### (2) Test design

A masking was done whereby the observers were unable to recognize the test drugs and the order of administration was made as random as possible. Numbers of the mice in one group was ten.

### (3) Method of the experiment

### 3-1) Test drugs

As representative examples of the benzene compounds (I) of the present invention, the following compounds were used. As the control compounds, morphine hydrochloride, WAY-100,635 disclosed in JP-A 5-170743 (EP 512755) and MDL-100,907 disclosed in US 5134149 were used.
(1) 1-[1-(4-Fluorophenethyl)piperidin-4-yl]-6-(N-acetylaminomethyl)indole;
(2) 1-[1-(4-fluorophenethyl)piperidin-4-yl]-6-(N-propionylaminomethyl)indole;
(3) 1-[1-(4-methoxyphenethyl)piperidin-4-yl]-6-(N-acetylaminomethyl)indole;
(4) 1-[1-(2-fluorophenethyl)piperidin-4-yl]-6-(N-acetylaminomethyl)indole;
(5) 1-[1-(4-fluorophenethyl)piperidin-4-yl]-6-(N-acetylamidemethyl)indole;
(6) 1-[1-(4-fluorophenethyl)piperidin-4-yl]-6-(N-hydroxyethylamidemethyl)indole;
(7) 1-[1-(4-fluorophenethyl)piperidin-4-yl]-6-(N,N-dimethylamidemethyl)indole oxalate;
(8) 1-[1-(4-fluorophenethyl)piperidin-4-yl]-6-amidemethylindole;
(9) 1- [1-(4-fluorophenethyl)piperidin-4-yl]-6-(N-methylamidemethyl)indole;
(10) 1-[1-(2-fluorophenethyl)piperidin-4-yl]-6-(N-methylamidemethyl)indole;
(11) 1-[1-(2-fluorophenethyl)piperidin-4-yl]-6-(N-ethylamidemethyl)indole;
(12) 1-[1-(2-fluorophenethyl)piperidin-4-yl]-6-(N-hydroxyethylamidemethyl)indole;
(13) 1-[1-(2-fluorophenethyl)piperidin-4-yl]-6-amidemethylindole;
(14) 1-[1-(4-fluorophenethyl)piperidin-4-yl]-6-(N-acetylaminomethyl)indoline;
(15) 1-(4-ethylpiperazin-l-yl)-3-(4-methoxyphenyl)isoquinoline;
(16) 1-(4-ethylpiperazin-1-yl)-3-(4-hydroxyethoxyphenyl)isoquinoline;
(17) 1-(4-ethylpiperazin-1-yl)-3-[4-(1-hydroxypropyl)phenyl]isoquinoline;
(18) 1- (4-ethylpiperazin-1-yl) -3- [4-(N-propylamide)phenyl]isoquinoline hydrochloride;
(19) 1-(4-ethylpiperazin-1-yl)-3-[3-fluoro-4-(3-hydroxy-3-methylbutyl)phenyl]isoquinoline hydrochloride;
(20) 1-(4-ethylpiperazin-1-yl)-3-(3-methoxy-4-hydroxypropylphenyl)isoquinoline hydrochloride;
(21) 1-(4-ethylpiperazin-1-yl)-3-(3-cyano-4-hydroxyethoxyphenyl)isoquinoline hydrochloride;
(22) 1-(4-ethylpiperazin-1-yl)-3-(3-hydroxypropylphenyl)isoquinoline hydrochloride;
(23) 1-(4-ethylpiperazin-1-yl)-3-(3-chloro-4-propylsulfonylaminomethylphenyl)isoquinoline hydrochloride;
(24) 4-(4-ethylpiperazin-1-yl)-6-[4-(2-hydroxypropoxy)phenyl]thieno[3,2-c]pyridine hydrochloride;
(25) 4-(4-ethylpiperazin-1-yl)-6-[4-(2-hydroxy-2-methylpropoxy)phenyl]thieno[3,2-c]pyridine hydrochloride;
(26) 4-(4-ethylpiperazin-1-yl)-6-[4-(1-hydroxyethyl)phenyl]thieno[3,2-c]pyridine oxalate; and
(27) 4-(4-hydroxyethylpiperazin-1-yl)-6-[4-(2-hydroxy-2-methylpropoxy)phenyl]thieno[3,2-c]pyridine hydrochloride.

### 3-2) Preparation of the test drugs

The compound to be tested was dissolved in a medium (a 5% sugar solution) on the day of the experiment, and then further diluted using the medium. In the case of a compound which is hardly soluble in the medium, the medium was added thereto followed by subjecting to an ultrasonic wave treatment so that the compound was dispersed, and then 1N hydrochloric acid was added thereto to dissolve.

Acetic acid was diluted with a physiological saline solution to prepare a 0.6% solution.

### 3-3) Schedule of the experiment

Body weight of the mouse fasted from the evening of the day before the experiment was measured, and then the mouse was transferred to each cage and acclimated for 30 minutes or longer. The test drug was subcutaneously administered (1 ml/100 g body weight) to the dorsal neck edge, then 0.6% acetic acid was intraperitoneally administered (1 ml/100 g body weight) after 15 minutes and the times of occurrence of the movement (writhing behavior) where the mouse extended the abdomen as if writhed during ten minutes thereafter were counted.

### 3-4) Analysis of data

Average writhing numbers and standard deviation were determined for each group. With regard to a statistic significance of the writhing behavior, a multiple comparative test of a Dunnett type (two-sided test; level of significance: 5%) was carried out after analysis of variance and the statistically minimum effective dose was determined.

### (4) Result

Analgesic action (minimum effective does) of the compounds of the present invention and of control drugs is shown in the following table.

There are also shown a serotonin (5HT) receptor binding action (RBA; *in vitro)* and a muscle relaxing action (*in vivo)* measured according to a method described in JP98/01481 or JP-A 10-281752. (In the table, "n.t." means "not tested").

| | Analgesic Action | RBA | | Muscle-Relaxing Action | |
|---|---|---|---|---|---|
| Compound | (mg/kg) | (Ki, nM) | | (mg/kg) | |
| | (sc) | 5HT1A | 5HT2 | (po) | (ip) |
| Morphine Hydrochloride | 1 | n.t. | n.t. | Ineffective (Strain Induced) | |
| WAY-100,635 | 3 | 0.4 | > 200 | n.t. | > 10 |
| MDL-100,907 | 3 | > 200 | 0.4 | 1 | 0.3 |
| (1) | 0.3 | 0.51 | 1.38 | 1 | 0.3 |
| (2) | 3 | 1.0 | 2.6 | 3 | 0.3 |
| (3) | 10 | 0.27 | > 20 | n.t. | n.t. |
| (4) | 3 | 1.58 | 0.75 | 10 | n.t. |
| (5) | 3 | 1.05 | 2.86 | 10 | n.t. |
| (6) | > 10 | 1.73 | 3.55 | 10 | n.t. |
| (7) | > 10 | 0.8 | 21.9 | n.t. | n.t. |
| (8) | > 3 | 0.06 | 3.29 | 3 | n.t. |
| (9) | 1 | 1.82 | 1.26 | 3 | 0.3 |
| (10) | 3 | 4.02 | 1.23 | < 1 | 3 |
| (11) | 3 | 1.78 | 1.64 | 10 | n.t. |
| (12) | 10 | 13.46 | 1.49 | > 10 | n.t. |
| (13) | 3 | 0.59 | 2.81 | 3 | 1 |
| (14) | 0.3 | 6.43 | 0.43 | 0.3 | 0.3 |
| (15) | 3 | 59.9 | 11.1 | n.t. | n.t. |
| (16) | 1 | 31.5 | 4.2 | 3 | 1 |
| (17) | 1 | 6.9 | 1.6 | 10 | 1 |
| (18) | 1 | 14.6 | 1.1 | 3 | > 10 |
| (19) | 1 | 31.2 | 0.7 | 3 | < 10 |
| (20) | 3 | 7.9 | 10.9 | 10 | n.t. |
| (21) | 1 | 13.4 | 4.5 | n.t. | 10 |
| (22) | 3 | 54.5 | 17.4 | n.t. | n.t. |
| (23) | 3 | 0.9 | 0.7 | > 30 | n.t. |
| (24) | 1 | 27.9 | 0.9 | 3 | 1 |
| (25) | 10 | 54.9 | 1.4 | 1 | 0.3 |
| (26) | 1 | 15.0 | 16.9 | < 3 | n.t. |
| (27) | 1 | 1530 | 0.35 | n.t. | n.t. |

Next, examples of the administration dosage form of the compound of the present invention are preparations for oral use such as powders, fine granules, granules, tablets, coated tablets and capsules; preparation for external use such as ointment and plasters; suppositories; and injections. In the manufacture of the preparations, conventional methods may be applied using common pharmaceutical carries.

That is, in the manufacture of the preparations for oral use, to the benzene compound (I) is added a filler followed, if necessary, by adding an antioxidant, a binder, a disintegrating agent, a lubricant, a coloring agent, a corrigent etc., and then the mixture is made into powders, fine granules, granules, tablets, coated tablets, capsules etc. by conventional means.

Examples of the filler are lactose, corn starch, sugar, glucose, mannitol, sorbitol, crystalline cellulose and silicon dioxide; examples of the binder are polyvinyl alcohol, polyvinyl ether, methyl cellulose, ethyl cellulose, gum arabic, tragacanth, gelatin, shellac, hydroxypropyl methylcellulose, hydroxypropyl cellulose, polyvinylpyrrolidone, polypropylene glycol-polyoxyethylene block polymer and meglumine; examples of the disintegrating agent are starch, agar, gelatin powder, crystalline cellulose, calcium carbonate, sodium hydrogen carbonate, calcium citrate, dextrin, pectin and calcium carboxymethyl cellulose; examples of the lubricant are talc, polyethylene glycol, silica and hydrogenated vegetable oil; examples of the coloring agent are those which are allowed to added to pharmaceuticals; and examples of the corrigent are cocoa powder, menthol, aromatic powder, peppermint oil, borneol and cinnamon powder. It is of course possible that those tablets and granules may be subjected to an appropriate coating by a sugar coat or others if necessary.

In the manufacture of preparations for injection, to the benzene compound (I) are added a pH adjusting agent, a solubilizer, an isotonizing agent etc. followed, if necessary, by adding solubilizing aid, a stabilizer, an antioxidant etc., and then the preparation is manufactured by a conventional method.

There is no particular limitation for the manufacture of the preparation for external use but the manufacture may be done by a conventional method. That is, as to the base materials for manufacturing the preparation, various ones which are commonly used for pharmaceuticals, pseudo-drugs, cosmetics, etc. may be used.

Specific examples of the base materials used therefor are animal and vegetable oils, mineral oil, ester oil, wax, higher alcohol, fatty acid, silicone oil, surface-active agents, alcohol, polyhydric alcohol, water-soluble high-molecular substances, clay minerals and purified water and, if necessary, pH adjusting agent, an antioxidant, a chelating agent, an antiseptic/antifungal agent, a coloring agent, perfume, etc. may be added as well although the base materials for the preparation for external use according to the present invention are not limited thereto. Further, if necessary, other components such as a blood flow promoter, a bactericide, an anti-inflammatory agent, a cell activator, vitamins, amino acid, a moisturizer and a keratin dissolving agent may be compounded therewith. The adding amount of the above-mentioned base material is that which gives the concentration usually set in the manufacture of the preparation for external use.

The clinical dose of the benzene compound (I) of the present invention is not limited but varies depending upon the diseases to be treated, the symptom, the degree of seriousness, the age, the complication etc., and also varies depending upon the administering route. Usually, however, the daily dose for an adult is 0.01mg to 2000 mg, preferably 0.1 mg to 1500 mg and more preferably, 1 mg to 1000 mg, and the administration is carried out orally, intravenously, intramuscularly, perrectally or percutaneously.

As fully illustrated hereinabove, it is clear that the benzene compounds (I) according to the present invention have quite excellent analgesic action which is same as or more than morphine hydrochloride. When the high safety of the benzene compounds (I) of the present invention is further taken into consideration, clinical usefulness of the present invention can be expected to be extremely excellent.

## Claims

1. An analgesic comprising the benzene compound (I) represented by the following formula: 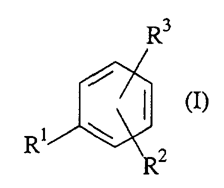 wherein, R¹ is a group represented by the following formula: 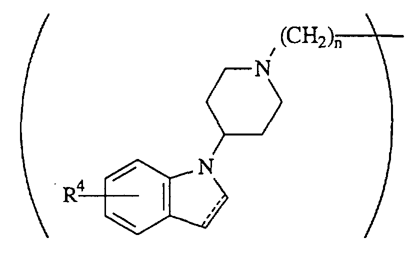 (wherein, R⁴ is a group selected from a lower acylaminoalkyl group, an amido lower alkyl group, an N-lower alkylamidoalkyl group, an N,N-di-lower alkylamidoalkyl group and an N-hydroxy lower alkylamidoalkyl group; n is an integer of 0 or 1 to 3; and the bond ----- means a single or double bond) or a group represented by the following formula: 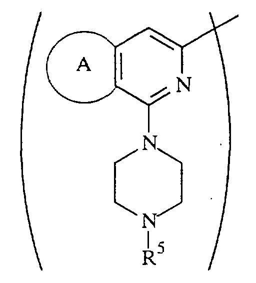 (in the formula, the ring A is benzene ring or thiophene ring; and R⁵ is a lower alkyl group or a hydroxy lower alkyl group); and R² and R³ are the same as or different from each other and each is a group selected from hydrogen atom, halogen atom, a lower alkyl group, a lower alkoxy group, a cyano group, a hydroxy lower alkyl group, a hydroxy lower alkoxy group, an N-lower alkylamide group and a lower alkylsulfonylaminoalkyl group, or a pharmacologically acceptable salt thereof as an active ingredient.

2. The analgesic according to claim 1 wherein the benzene compound (I) is a compound represented by the following formula (II) or (III): 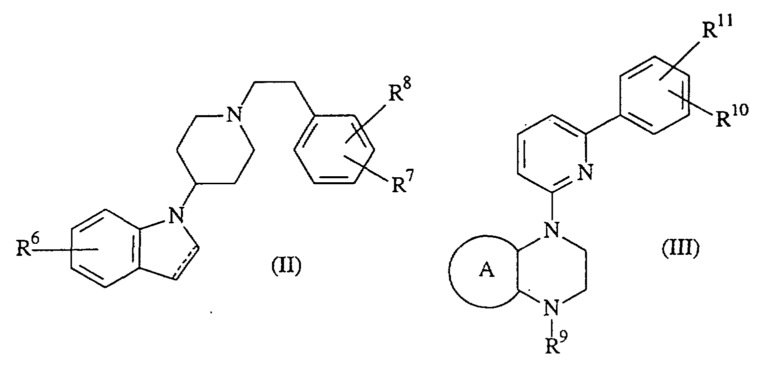 (wherein, R⁶ has the same meaning as the above-mentioned R⁴; and R⁷, R⁸, R¹⁰ and R¹¹ have the same meanings as the above-mentioned R² and R³; the ring A has the same meaning as mentioned above; and the bond ----- means a single or double bond), or a pharmacologically acceptable salt thereof.

3. The analgesic according to claim 1 or 2 wherein the benzene compound (I) is the one selected from the following compounds:
(1) 1-[1-(4-fluorophenethyl)piperidin-4-yl]-6-(N-acetylaminomethyl)indole;
(2) 1-[1-(4-fluorophenethyl)piperidin-4-yl]-6-(N-propionylaminomethyl)indole;
(3) 1-[1-(4-methoxyphenethyl)piperidin-4-yl]-6-(N-acetylaminomethyl)indole;
(4) 1-[1-(2-fluorophenethyl)piperidin-4-yl]-6-(N-acetylaminomethyl)indole;
(5) 1-[1-(4-fluorophenethyl)piperidin-4-yl]-6-(N-acetylamidemethyl)indole;
(6) 1- [1-(4-fluorophenethyl)piperidin-4-yl]-6-(N-hydroxyethylamidemethyl)indole ;
(7) 1-[1-(4-fluorophenethyl)piperidin-4-yl]-6-(N,N-dimethylamidemethyl)indole;
(8) 1-[1-(4-fluorophenethyl)piperidin-4-yl]-6-amidemethylindole;
(9) 1-[1-(4-fluorophenethyl)piperidin-4-yl]-6-(N-methylamidemethyl)indole;
(10) 1-[1-(2-fluorophenethyl)piperidin-4-yl]-6-(N-methylamidemethyl)indole;
(11) 1-[1-(2-fluorophenethyl)piperidin-4-yl]-6- (N-ethylamidemethyl)indole;
(12) 1-[1-(2-fluorophenethyl)piperidin-4-yl] -6- (N-hydroxyethylamidemethyl)indole;
(13) 1-[1-(2-fluorophenethyl)piperidin-4-yl]-6-amidemethylindole;
(14) 1- [1-(4-fluorophenethyl)piperidin-4-yl]-6-(N-acetylaminomethyl)indoline;
(15) 1-(4-ethylpiperazin-1-yl)-3-(4-methoxyphenyl)isoquinoline;
(16) 1- (4-ethylpiperazin-1-yl) -3- (4-hydroxyethoxyphenyl)isoquinoline;
(17) 1-(4-ethylpiperazin-1-yl)-3-[4-(1-hydroxypropyl) phenyl]isoquinoline;
(18) 1- (4-ethylpiperazin-1-yl)-3-[4-(N-propylamide)phenyl]isoquinoline;
(19) 1-(4-ethylpiperazin-1-yl)-3-[3-fluoro-4-(3-hydroxy-3-methylbutyl)phenyl]isoquinoline;
(20) 1-(4-ethylpiperazin-1-yl)-3-(3-methoxy-4-hydroxypropylphenyl)isoquinoline;
(21) 1-(4-ethylpiperazin-1-yl)-3-(3-cyano-4-hydroxyethoxyphenyl)isoquinoline;
(22) 1-(4-ethylpiperazin-1-yl)-3-(3-hydroxypropylphenyl)isoquinoline;
(23) 1-(4-ethylpiperazin-1-yl)-3-(3-chloro-4-propylsulfonylaminomethylphenyl)isoquinoline;
(24) 4- (4-ethylpiperazin-1-yl)-6-[4-(2-hydroxypropoxy)phenyl]thieno[3,2-c]pyridine;
(25) 4-(4-ethylpiperazin-1-yl)-6-[4-(2-hydroxy-2-methylpropoxy)phenyl]thieno[3,2-c]pyridine;
(26) 4-(4-ethylpiperazin-1-yl)-6-[4-(1-hydroxyethyl)phenyl]thieno[3,2-c]pyridine; and
(27) 4-(4-hydroxyethylpiperazin-1-yl)-6-[4-(2-hydroxy-2-methylpropoxy)phenyl]thieno[3,2-c]pyridine.

4. The analgesic according to any of claims 1 to 3, which prevents, treats or improves headache or migraine.

5. A method for preventing, treating or improving headache and migraine, by administering a pharmacologically effective dose of the benzene compound (I) defined in claim 1 or a pharmacologically acceptable salt thereof to a person.

6. Use of the benzene compound (I) defined in claim 1 or a pharmacologically acceptable salt thereof for producing analgesics.
